## Europäisches Patentamt

## European Patent Office

## Office européen des brevets

(19)

(11) Numéro de publication: **0 149 388**

**B1**

(12)

# FASCICULE DE BREVET EUROPÉEN

(45) Date de publication du fascicule du brevet:
**12.08.87**

(21) Numéro de dépôt: **84402584.1**

(22) Date de dépôt: **13.12.84**

(51) Int. Cl.⁴: **C 07 C 119/048,** C 07 C 118/02,
C 07 C 118/00, C 08 G 18/76

(54) Composition d'isocyanates de bas point de fusion à structure diphényléthane, son procédé de fabrication; application à la fabrication de polyuréthannes.

(30) Priorité: **21.12.83 FR 8320458**

(43) Date de publication de la demande:
**24.07.85 Bulletin 85/30**

(45) Mention de la délivrance du brevet:
**12.08.87 Bulletin 87/33**

(84) Etats contractants désignés:
**BE CH DE FR GB IT LI NL**

(56) Documents cité:
**EP-A-0 046 556**
**EP-A-0 107 577**
**FR-A-1 492 642**

**CHEMICAL ABSTRACTS, vol. 65, no. 4, 15 août 1966,**
**colonne 5421e, Columbus, Ohio, US**

(73) Titulaire: **ATOCHEM, 4 & 8, Cours Michelet La
Défense 10, F-92800 Puteaux (FR)**

(72) Inventeur: **Kervennal, Jacques, 134, rue E. Locard,
F-69005 Lyon (FR)**
Inventeur: **Mathais, Henri, Hameau de St Didier
No 2 Chemin de l'Indiennerie, F-69370 Saint-
Didier- Au- Mont- d'Or (FR)**

(74) Mandataire: **Foiret, Claude, ATOCHEM
Département Propriété Industrielle, F-92091 Paris
la Défense 10 Cédex 42 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 149 388 B1

## Description

L'invention concerne une nouvelle composition d'isocyanates, contenant des diisocyanates à structure diphényléthane et obtenue à partir de diphényléthane par dinitration des noyaux aromatiques, suivie d'une hydrogénation et d'une phosgénation. Cette composition possède un point de fusion abaissé par rapport aux diisocyanates de bi-benzyle déjà connus dans la littérature; de plus elle a la propriété de rester plusieurs heures en surfusion, ce qui facilite son utilisation, notamment dans l'élaboration de polyuréthannes. La répartition en poids des diisocyanates dans cette composition est de 35 à 55 % d'isomère 2,4', de 15 à 40 % d'isomère 4,4', de 10 à 25 d'isomère 2,2', de 3 à 10 % d'isomère 3,4' et de 3 à 8 % d'isomère 2,3'.

Des diisocyanates à structure aromatique hydrocarbonée diphénylméthane sont synthétisés industriellement (M.D.I). Ils sont obtenus par condensation de deux molécules d'aniline sur une de formol en présence d'un catalyseur acide, suivie d'une phosgénation, ce qui conduit à des mélanges contenant les isomères 4,4', 2,4' et 2,2'. Mais cette condensation n'est jamais totalement sélective en produits à 2 noyaux et on synthétise habituellement un mélange de polyisocyanates et de diisocyanates, ces derniers étant séparés par distillation pour donner du MDI pur. L'isomère MLI-4,4' est en général nettement majoritaire et possède un point de fusion de 38°C, ainsi, suivant sa teneur, il peut être nécessaire de liquéfier le mélange de diisocyanates avant de pouvoir l'utiliser, soit en le fondant et en travaillant à chaud, soit en le transformant chimiquement.

Des isocyanates à structure diphényléthane ont déjà été décrits dans la littérature: il s'agit de diisocyanates 4,4' diphényléthane dont la préparation a été publiée par phosgénation de la diamine correspondante par E.COCEA; A.CARACULACU; C.MARCULESCU; A.PETRUS; I.MATEI dans Studii si Cercetàri Stiintifice - Chimie. Academia R.P. Române Filiala Iasi (1959), 10, n°2, p. 261. La phosgénation a également été décrite dans le brevet japonais 41-6583. On obtient ainsi, après distillation, un diisocyanate ayant un point de fusion de 88-89°C, ce qui nécessite de travailler à température élevée pour fabriquer des polyuréythannes.

Quelques publications traitent des applications de ce diisocyanate : I.MATEI; E.COCEA; A.CARACULACU; A.PETRUS dans Studii si Cercetàri Stiintifice-Chimie. Academia R.P. Române Filiala Iasi (1960), 379 et D.J.LYMAN ; J.HELLER ; M.BARLOW dans Die Makromolekulare Chemie 84, p.64 (1965) ont décrit la synthèse et certaines propriétés de polyuréthannes dérivés du diisocyanate-4,4' diphényléthane.

L'isomère diisocyanate-2,2' diphényléthane a été aussi cité dans le brevet français 1 492 642, mais son point de fusion 78-79°C est également élevé. On peut le préparer par duplication oxydante de l'orthonitrotoluène, suivie d'une hydrogénation puis d'une phosgénation; cependant le rendement de la première étape est médiocre.

Si on réalise la duplication oxydante en partant d'un mélange des isomères de nitrotoluène, on obtient un mélange des isomères dinitrés qu'il est possible d'hydrogéner et des phosgéner. Mais là encore le rendement de la première étape est médiocre et c'est l'isomère 4,4' qui est favorisé, on obtient donc un mélange des diisocyanates où cet isomère est majoritaire et qui ne présente pas les propriétés de surfusion des compositions conformes à l'invention.

Les mélanges conformes à l'invention ont l'avantage d'avoir un point de fusion abaissé par rapport aux isomères purs cités précédemment. De plus, ils présentent la particularité, une fois fondus, de demeurer plusieurs heures en surfusion, ce qui permet de les mettre en oeuvre à l'etat liquide, à temperature ordinaire. Par ailleurs, le fait d'obtenir des mélanges d'isomère permet de disposer de groupements isocyanates de réactivités variables.

La synthèse des isocyanates selon l'invention fait appel à trois étapes réactionnelles successives à partir de diphényléthane: une de nitration des noyaux aromatiques, une d'hydrogénation et une de phosgénation. Le diphényléthane utilisé comme matière première peut être préparé par duplication oxydante du toluène comme l'ont décrit LIU K.H.D. et YAMASAKI Y. dans Bull. Jpn. Pet. Inst. (1976), 18, 45 ou par condensation du type FRIEDEL et CRAFTS du dichloroéthane sur le benzène ainsi que l'ont publie SHUZO Y. et TAKAO H. dans J. Soc. Chem. Ind. Japan 47, 814 51944).

On utilise pour la nitration un mélange d'acide nitrique et d'acide sulfurique concentrés à au moins 90 % en poids, un plus grand excès entraînant une formation prohibitive de dérivés trinitrés. L'acide nitrique peut être placè en quantités stoechiométriques par rapport au composé aromatique utilisant ainsi une mole d'acide par noyau aromatique à nitrer. Il est cependant avantageux, d'opérer en présence d'un excès d'acide nitrique pouvant aller jusqu'à 20 % par rapport à la stoechiométrie, une concentration inférieure entraînant une formation prohibitive de dérivés mononitrés. L'acide sulfurique peut être utilisé en quantité équimolaire par rapport à l'acide nitrique, mais on peut le placer en excès ou en défaut. La réaction de nitration peut s'effectuer entre 0°C et la température d'ébullition des mélanges, habituellement entre 0°C et 50°C, le composé aromatique étant de préférence solubilisé dans un solvant tel que le chlorure de méthylène. L'addition du mélange d'acides peut donc se faire à 0°C, mais il n'est pas gênant d'opérer à température ambiante à condition de contrôler l'exothermicité de la réaction. Celle-ci peut se poursuivre à température ambiante, mais il est préférable d'opérer au reflux du mélange, ce qui permet d'éliminer en partie l'excès d'acide nitrique. La phase organique, supérieure, est ensuite séparée des acides, neutralisée et évaporée à sec. Il est recommandé d'opérer sous atmosphère d'azote, dans un réacteur muni de moyens d'agitation efficace et de régulation de température.

En suivant le mode opératoire ainsi décrit, on obtient, à partir de diphényléthane pur, des produits formés essentiellement d'isomères de dinitration, contenant de 35 à 55 % de dinitro-2,4' diphényléthane ; de 15 à 40 % d'isomère 4,4' ; de 10 à 25 % d'isomère 2,2' ; de 3 à 10 % d'isomère 3,4' et de 3 à 8 % d'isomère 2,3'.

La deuxième étape constitue l'hydrogénation des dérivés nitrés en amines correspondantes; elle peut être chimique, mais il est préférable d'opérer sous pression d'hydrogène, dans un réacteur résistant à la pression, muni d'une agitation et possédant les moyens de contrôle et régulation classiques, en présence d'un catalyseur à base de nickel, palladium, platine , ruthénium et autres.

Dans ce cas, on utilise un réacteur d'hydrogénation permettant de travailler sous des pressions pouvant atteindre 100 bars. La réaction peut s'effectuer sans solvant à une température où le dérivé nitré est fondu, ou dans des solvants classiques d'hydrogénation tels que les alcools, le dioxane, les éthers de l'éthylèneglycol et autres.

On utilise préférentiellement un catalyseur constitué de palladium déposé sur support à des teneurs comprises entre 1 et 10 % ce qui permet d'opérer à des températures comprises entre 30 et 100° C et des pressions de 20 à 50 bars. Le rapport molaire dérivés nitrés n'est pas impérativement fixé, mais il est de palladium préférence compris entre 200 et 3 000. Après réaction et filtration du catalyseur, le solvant éventuellement utilisé est évaporé et le mélange d'amines obtenu peut être utilisé tel quel. Il est aussi possible de séparer à ce stade les isomères de diamino-diphényléthane par distillation ou par recristallisation.

La troisième étape fait appel à des techniques classiques de phosgénation dans un réacteur muni d'une agitation et surmonté d'un réfrigérant. Par exemple, le mélange d'amines est placé à une concentration de 5 à 20 % dans un solvant aromatique chloré tel que le monochlorobenzène ou l'orthodichlorobenzène contenant la quantité nécessaire de phosgène, en maintenant la température aux environs de 20° C. On chauffe ensuite la suspension progressivement. Le mélange réactionnel s'homogénéise vers 100° C. On continue d'élever lentement la température jusqu'au point d'ébullition du mélange, puis on distille le solvant de façon à récupérer les isocyanates formés.

Une autre technique consiste à introduire conjointement dans le réacteur chauffé au-dessus de 80° C, une solution des isomères de diamines dans le solvant choisi et un courant de phosgène gazeux en léger excès. La formation des isocyanates est alors pratiquement immédiate.

On peut également former dans un premier temps les chlorhydrates des amines, puis faire réagir ceux-ci avec un courant de phosgène gazeux à une température supérieure à 120° C.

Dans tous les cas, le solvant est ensuite distillé de façon à récupérer les isocyanates bruts formés. Ces derniers pouvant être utilisés tels quels par exemple dans certaines formulations de polyuréthannes. Cependant, les isomères de diisocyanates peuvent etre distillés sous un vide poussé de façon à obtenir la composition de l'invention sous forme pure.

Les dérivés nitrés ont été analysés par chromatographie en phase gazeuse (CPV) en utilisant une colonne en pyrex de diamètres intérieur et extérieur 3 et 6 mm, de longueur 2 mètres, remplie de phase SP 2250 imprégnée à 3 % sur support Supelcoport 100-120 mesh (SUPELCO) sur laquelle on a effectué des programmations linéaires de température à 40° C/mn de 180° C à 280° C. Les isomères dinitrés principaux ont été synthétisés purs ou isolés par piégeage et identifiés par resonnance magnétique nucléaire du $^{13}$C et du $^{1}$H.

Pour les amines, analysées également par chromatographie en phase gazeuse, on utilise une colonne en verre de 2 mètres, emplie de support chromosorb W.N.A.W. 60-80 mesh (Johns MANVILLE), imprégnée à 5 % de KOH et 5 % d'Apièzon N (Société Apièzon Products Limited), en opérant en isotherme à 220° C. Les teneurs en fonctions aminées totales sont dosées chimiquement. Les fonctions NCO des isocyanates sont évaluées par dosage chimique, tandis que les compositions en isomères sont déterminées par chromatographie en phase gazeuse sur la colonne et dans les conditions utilisées pour les dérivés nitrés.

## EXEMPLE 1

Dans un réacteur de 6 litres, on dissout 500 g de diphényléthane (2,74 moles) dans 1,1 litre de chlorure de méthylène. On introduit à température ambiante un mélange de 387,5 g d'acide nitrique à 98,7 % (excès de 10 % par rapport à la stoéchiométrie) et de 750 g d'acide sulfurique à 96 % (excès de 34 %). L'addition dure 45 minutes, puis on porte le mélange à reflux pendant 3 H 30. Après refroidissement, on ajoute 2,2 litres de chlorure de méthylène et laisse décanter. On extrait la phase organique avec deux fractions complémentaires de 1,5 l de chlorure de méthylène, puis on neutralise à l'aide de carbonate de soude et filtre. Après évaporation du solvant, on récupère 730 g d'un produit solide blanc-jaune fondant à 140° C. L'analyse par C.P.V. donne les proportions suivantes d'isomères : 41,8 % de 2,4' ; 32,4 % de 4,4' ; 13,2 % de 2,2' ; 7,5 % de 3,4' et 4,5 % de 2,3'. On ne détecte ni mono, ni trinitrés.

On charge dans un autoclave à balancelle de 5 litres 375 g du produit récupéré ci-dessus qu'on met en suspension dans 3,5 litres de méthanol. On ajoute 3,5 g de catalyseur palladium déposé à 5 % sur du charbon et isole l'autoclave après l'avoir balayé à l'azote. On introduit 40 bars d'hydrogène et chauffe à 100-120°C pendant 1 heure. Après refroidissement on récupère le mélange, le filtre et évapore le méthanol. On recueille 288 g du mélange des diamines de diphényléthane. Le reste des dérivés nitrés est hydrogéné de la même façon.

On phosgène ensuite de la façon suivante: dans un réacteur de 6 litres, on introduit 3,6 litres d'orthodichlorobenzène qu'on refroidit à 5°C. On coule alors 1,07 kg de phosgène liquide puis introduit 503 g du mélange de diamines. Il se forme un précipité de chlorure de carbamoyle. On laisse réagir 2 heures à température ambiante, 1 heure à 45°C, 2 heures à 60°C et 1/2 heure à 100°C. A cette température, le mélange devient limpide. On chauffe jusqu'à 140°C puis introduit un balayage d'azote jusqu'à élimination totale du phosgène. Après refroidissement, on filtre puis évapore le solvant. Le résidu de 614 g constitué des isomères d'isocyanates est distillé sous pression réduite de 266 Pa. dans un appareillage formé d'un ballon, chauffé entre 230 et 260°C, surmonté d'un pont de distillation. Le mélange purifié des diisocyanates contient 2 équivalents NCO par mole et se présente sous forme d'un liquide qui se solidifie au bout de plusieurs heures. Le mélange possède alors un point de fusion complète de 50°C et demeure quelques heures à l'état liquide, ce qui permet de le manipuler facilement.

Sa composition est sensiblement la suivante:

isomère 2,4' : 45,0 %
isomère 4,4' : 30,3 %
isomère 2,2' : 15,0 %
isomère 3,4' : 5,0 %
isomère 2,3' : 4,7 %

## EXEMPLE 2

On nitre 18,2 g de diphényléthane en opérant comme dans l'exemple 1, mais en utilisant 16,6 g d'acide nitrique à 98,7 % (excès de 30 %) et 26,5 g d'acide sulfurique à 96 % (excès de 30 %). On récupère en fin de réaction, après distillation du chlorure de méthylène, 25 g d'un mélange de dérivés nitrés contenant 10,9 % de trinitro-diphényléthanes conduisant après hydrogénation et phosgénation, en excluant les triisocyanates formés, un mélange de diisocyanates de composition voisine de:

isomère 2,4' : 38 %
isomère 4,4' : 40 %
isomère 2,2' : 11 %
isomère 3,4' : 7 %
isomère 2,3' : 4 %

## EXEMPLE 3

On nitre 18,2 g de diphényléthane en opérant comme dans l'exemple 1, mais en utilisant 15,4 g d'acide nitrique à 90 % (excès de 10 %) et 25,5 g d'acide sulfurique à 90 % (excès de 30 %). On obtient en fin de réaction, 24,5 g d'un mélange de dérivés nitrés contenant 8,7 % de mononitrodiphényléthanes conduisant après hydrogénation et phosgénation, en excluant les monoisocyanates formés, à un mélange de diisocyanates de compostion voisine de:

isomère 2,4' : 42 %
isomère 4,4' : 34 %
isomère 2,2' : 13 %
isomère 3,4' : 7 %
isomère 2,3' : 4 %

## EXEMPLE 4 (exemple comparatif)

On réalise la duplication oxydante du paranitrotoluène en dinitro-4,4' bibenzyle suivant la description faite par H.A. STANSBURY Jr et W.A. PROOPS dans le J.Org. Chem. Vol. 26, p.4162 (1961). Pour cela, on place dans un réacteur refroidi à 5°C et muni d'une agitation efficace, 93 g de solution de potasse méthanolique à 28 %. On fait passer un courant d'oxygène et introduit lentement 27,4 g de paranitrotoluène dissous dans 60 g d'éthylène diamine à 99 %, en contrôlant la température en dessous de 10°C. On laisse réagir pendant 1 heure, puis dilue avec 160 ml d'eau, filtre et lave le précipité à l'eau et au méthanol. On récupère après séchage 26 g de dinitro-4,4' bibenzyle contenant des traces de dinitro-4,4' stilbène (taux de transformation : 95 %).

On hydrogène 10 g de ce produit dans un autoclave de 300 ml en opérant dans 100 ml de méthanol en présence de 0,2 g de catalyseur palladium sur charbon à 5 %, l'autoclave est porté à 120°C sous 30 bars d'hydrogène jusqu'à ce qu'on ne détecte plus de chute de pression. Après refroidissement, filtration du catalyseur et évaporation du solvant, on récupère 7,7 g de diamino-4,4' diphényléthane, de pureté 98-99 %, ayant un point de fusion de 137°C.

La phosgénation est effectuée selon le mode opératoire décrit dans l'exemple 1. On récupère, après distillation sous pression réduite, le diisocyanate-4,4' diphényléthane contenant 2 équivalents NCO par mole et ayant un point de fusion de 88-89°C.

## EXEMPLE 5 (exemple comparatif)

On effectue la duplication oxydante de 13,7 g d'orthonitrotoluène en dinitro-2,2' diphényléthane selon la technique décrite dans l'exemple 4. On récupère 6,8 g du dérivé dinitré (taux de transformation : 50 %). L'hydrogénation de 10 g du dérivé dinitré est effectuée comme dans l'exemple 4 et conduit à 7,8 g de diamino-2,2' bibenzyle fondant à 76° C. La phosgénation de la diamine selon le mode opératoire décrit dans l'exemple 1 fournit le diisocyanate-2,2' diphényléthane qui, distillé, contient 2 équivalents NCO par mole et a un point de fusion de 78-79° C.

**EXEMPLE 6** (exemple comparatif)

On effectue la réaction de duplication oxydante sur un mélange de 18 g d'orthonitrotoluène et de 10 g de paranitrotoluène, en opérant selon la technique décrite dass l'exemple 4. On récupère ainsi 10 g de produit orangé solide formé des isomères 4,4', 2,2' et 2,4' de dinitrodiphényléthane. Après hydrogénation de 9,5 g du mélange et phosgénation dans les conditions décrites dans les exemples précédents, on recueille, après distillation du solvant, 7,2 g d'isocyanate solide brun fondant à 60° C, contenant 1,90 équivalent NCO/mole et dont la répartition des isomères est la suivante : 74,7 % de 4,4'; 21,3 % de 2,2'; 4 % de 2,4'.

**EXEMPLE 7**

On place à 50° C dans un réacteur 100 parties de polyadipate de butanediol, 20 parties de butanediol et 3 à 4 gouttes d'une diamine tertiaire (commercialisée sous le nom de DABCO). On coule à 50° C un mélange des diisocyanates de diphényléthane préparé dans l'exemple de façon à avoir un rapport $\frac{NCO}{OH}$ = 1. On agite pendant 15 secondes puis coule le mélange réactionnel dans un moule qu'on place pendant 5 heures à 110° C. On obtient alors une plaque semi-rigide sur laquelle ont été effectuées quelques mesures :

. la dureté shore D est de 30
. essais de traction (avec une vitesse de traction de 50mm/mn)
. charge de rupture (Mpa)      : 23,4
. allongement correspondant (%) : 672
. charge maximum (Mpa)      : 25,6
. allongement correspondant (%) : 668

**Revendications**

1. Composition de bas point de fusion contenant des diisocyanates de diphényléthane dont la répartion en poids est de:

35 à 55 % d'isomère 2,4'
15 à 40 % d'isomère 4,4'
10 à 25 % d'isomère 2,2'
3 à 10 % d'isomère 3,4'

3 à 8 % d'isomère 2,3'

2. Procédé de fabrication de la composition selon la revendication 1 caractérisé en ce qu'elle est obtenue par nitration des noyaux aromatiques du diphényléthane, suivie d'une hydrogénation et d'une phosgénation.

3. Procédé de fabricatios selon la revendication 2 caractérisé es ce que la nitratios s'effectue au moyen d'us mélange d'acide nitrique et d'acide sulfurique concentrés à au moins 90 % en poids.

4. Procédé selon l'une des revendications 2 à 3 caractérisé es ce que la nitration s'effectue en présence d'un excès d'acide nitrique n'excédant pas 20 % par rapport à la stoechiométrie.

5. Application de la composition selon la revendication 1 à la fabrication de polyuréthannes.

**Claims**

1. Low melting point composition containing diphenylethane diisocyanates, the distribution of which by weight is as follows:

35 to 55% of 2,4'-isomer
75 to 40 % of 4,4'-isomer
10 to 25 % of 2,2'-isomer
3 to 10 % of 3,4'-isomer
3 to 8 % of 2,3'-isomer

2. Process for manufacturing the composition according to Claim 1, characterized in that it is obtained by nitration of the aromatic rings of diphenylethane, followed by hydrogenation and phosgenation.

3. Manufacturing process according to Claim 2, characterized in that the nitration is performed by means of a mixture of concentrated nitric acid and concentrated sulphuric acid containing at least 90 % by weight.

4. Process according to one of Claims 2 and 3, characterized in that the nitration is performed in the presence of an excess of nitric acid not exceeding 20 % with respect to the stoichiometric amount.

5. Application of the composition according to Claim 1 to the manufacture of polyurethanes.

**Patentansprüche**

1. Mittel mit niederem Schmelzpunkt, enthaltend Diphenylethan-diisocyanate mit folgender gewichtsmäßigen Verteilung:

35 bis 55 % 2,4'-Isomer
15 bis 40 % 4,4'-Isomer
10 bis 25 % 2,2'-Isomer
3 bis 10 % 3,4'-Isomer
3 bis 8 % 2,3'-Isomer.

2. Verfahren zur Herstellung des Mittels nach

Anspruch 1,
dadurch gekennzeichnet , daß es durch Nitrierung der aromatischen Kerne des Diphenylethans und anschließende Hydrierung sowie Phosgenierung erhalten wird.

3. Herstellungsverfahren nach Anspruch 2, dadurch gekennzeichnet , daß die Nitrierung mit Hilfe eines Gemisches aus (jeweils) konzentrierter, mindestens 90 gew.-%iger Salpetersäure und Schwefelsäure vorgenommen wird.

4. Verfahren nach einem der Ansprüche 2 bis 3, dadurch gekennzeichnet , daß die Nitrierung in Anwesenheit eines Überschusses von nicht mehr als 20 % Salpetersäure, bezogen auf das stöchiometrische Verhältnis, durchgeführt wird.

5. Anwendung des Mittels nach Anspruch 1 zur Herstellung von Polyurethanen.